# EUROPEAN PATENT APPLICATION

(11) **EP 1 437 411 A1**
(43) Date of publication of application: **14.07.2004**
(21) Application number: 02772933.4
(22) Date of filing: 26.09.2002
(51) Int. Cl.: C12N 15/53, C12N 9/04, C12N 1/15, C12N 1/19, C12N 1/21, C12N 5/00, C12P 21/02, G01N 33/48

(54) **GLUCOSE DEHYDROGENASE**

(30) Priority: 26.09.2001 JP 2001294846
(71) Applicant: Sode, Koji, Tokyo 152-0013 (JP)
(72) Inventor: Sode, Koji, Tokyo 152-0013 (JP)
(74) Representative: VOSSIUS & PARTNER
(86) International application number: PCT/JP2002/009943
(87) International publication number: WO 2003/027294

(57) **Abstract**

A modified glucose dehydrogenase is disclosed which comprises a water-soluble glucose dehydrogenase having a pyrroloquinoline quinone as a coenzyme. At least one amino acid residue present on the surface of the water-soluble glucose dehydrogenase is replaced with arginine. The side chain of the amino acid residue is exposed at the surface of the molecule and is not expected to substantially interact with other residues. The amino acid residue is present in a region that is probably not an enzyme active site or a substrate binding site. Preferably the amino acid residue is selected from the group consisting of glutamine, asparagine, and threonine. This modified enzyme can be prepared by recombinant processes and recovered efficiently.

Sequence Listing
<110> Sode, Koji
<120> Glucose Dehydrogenase
<140> PCT/JP02/09943
<141> 2002-09-26
<150> JP 2001-294846
<151> 2001-09-26
<160> 5
<210> 1
<211> 454
<212> PRT
<213> Acinetobacter calcoaceticus
<400> 1
Asp Val Pro Leu Thr Pro Ser Gln Phe Ala Lys Ala Lys Ser Glu Asn
1               5                   10                  15
Phe Asp Lys Lys Val Ile Leu Ser Asn Leu Asn Lys Pro His Ala Leu
            20                  25                  30
Leu Trp Gly Pro Asp Asn Gln Ile Trp Leu Thr Glu Arg Ala Thr Gly
        35                  40                  45
Lys Ile Leu Arg Val Asn Pro Glu Ser Gly Ser Val Lys Thr Val Phe
    50                  55                  60
Gln Val Pro Glu Ile Val Asn Asp Ala Asp Gly Gln Asn Gly Leu Leu
65                  70                  75                  80
Gly Phe Ala Phe His Pro Asp Phe Lys Asn Asn Pro Tyr Ile Tyr Ile
                85                  90                  95
Ser Gly Thr Phe Lys Asn Pro Lys Ser Thr Asp Lys Glu Leu Pro Asn
            100                 105                 110
Gln Thr Ile Ile Arg Arg Tyr Thr Tyr Asn Lys Ser Thr Asp Thr Leu
        115                 120                 125
Glu Lys Pro Val Asp Leu Leu Ala Gly Leu Pro Ser Ser Lys Asp His
    130                 135                 140
Gln Ser Gly Arg Leu Val Ile Gly Pro Asp Gln Lys Ile Tyr Tyr Thr
145                 150                 155                 160
Ile Gly Asp Gln Gly Arg Asn Gln Leu Ala Tyr Leu Phe Leu Pro Asn
                165                 170                 175
Gln Ala Gln His Thr Pro Thr Gln Gln Glu Leu Asn Gly Lys Asp Tyr
            180                 185                 190
His Thr Tyr Met Gly Lys Val Leu Arg Leu Asn Leu Asp Gly Ser Ile
        195                 200                 205
Pro Lys Asp Asn Pro Ser Phe Asn Gly Val Val Ser His Ile Tyr Thr
    210                 215                 220
Leu Gly His Arg Asn Pro Gln Gly Leu Ala Phe Thr Pro Asn Gly Lys
225                 230                 235                 240
Leu Leu Gln Ser Glu Gln Gly Pro Asn Ser Asp Asp Glu Ile Asn Leu
                245                 250                 255
Ile Val Lys Gly Gly Asn Tyr Gly Trp Pro Asn Val Ala Gly Tyr Lys
            260                 265                 270
Asp Asp Ser Gly Tyr Ala Tyr Ala Asn Tyr Ser Ala Ala Ala Asn Lys
        275                 280                 285
Ser Ile Lys Asp Leu Ala Gln Asn Gly Val Lys Val Ala Ala Gly Val
    290                 295                 300
Pro Val Thr Lys Glu Ser Glu Trp Thr Gly Lys Asn Phe Val Pro Pro
305                 310                 315                 320
Leu Lys Thr Leu Tyr Thr Val Gln Asp Thr Tyr Asn Tyr Asn Asp Pro
                325                 330                 335
Thr Cys Gly Glu Met Thr Tyr Ile Cys Trp Pro Thr Val Ala Pro Ser
            340                 345                 350
Ser Ala Tyr Val Tyr Lys Gly Gly Lys Lys Ala Ile Thr Gly Trp Glu
        355                 360                 365
Asn Thr Leu Leu Val Pro Ser Leu Lys Arg Gly Val Ile Phe Arg Ile
    370                 375                 380
Lys Leu Asp Pro Thr Tyr Ser Thr Thr Tyr Asp Asp Ala Val Pro Met
385                 390                 395                 400
Phe Lys Ser Asn Asn Arg Tyr Arg Asp Val Ile Ala Ser Pro Asp Gly
                405                 410                 415
Asn Val Leu Tyr Val Leu Thr Asp Thr Ala Gly Asn Val Gln Lys Asp
            420                 425                 430
Asp Gly Ser Val Thr Asn Thr Leu Glu Asn Pro Gly Ser Leu Ile Lys

435                 440                 445
Phe Thr Tyr Lys Ala Lys
    450
<210> 2
<211> 1612 <212> DNA <213> Acinetobacter calcoaceticus
<400> 2 agctactttt atgcaacaga gcctttcaga aatttagatt ttaatagatt cgttattcat 60
cataatacaa atcatataga gaactcgtac aaacccttta ttagaggttt aaaaattctc 120
ggaaaatttt gacaatttat aaggtggaca catgaataaa catttattgg ctaaaattgc 180
tttattaagc gctgttcagc tagttacact ctcagcattt gctgatgttc ctctaactcc 240
atctcaattt gctaaagcga aatcagagaa ctttgacaag aaagttattc tatctaatct 300
aaataagccg catgctttgt tatggggacc agataatcaa atttggttaa ctgagcgagc 360
aacaggtaag attctaagag ttaatccaga gtcgggtagt gtaaaaacag tttttcaggt 420
accagagatt gtcaatgatg ctgatgggca gaatggttta ttaggttttg ccttccatcc 480
tgattttaaa aataatcctt atatctatat ttcaggtaca tttaaaaatc cgaaatctac 540
agataaagaa ttaccgaacc aaacgattat tcgtcgttat acctataata aatcaacaga 600
tacgctcgag aagccagtcg atttattagc aggattacct tcatcaaaag accatcagtc 660
aggtcgtctt gtcattgggc cagatcaaaa gatttattat acgattggtg accaagggcg 720
taaccagctt gcttatttgt tcttgccaaa tcaagcacaa catacgccaa ctcaacaaga 780
actgaatggt aaagactatc acacctatat gggtaaagta ctacgcttaa atcttgatgg 840
aagtattcca aaggataatc caagttttaa cggggtggtt agccatattt atacacttgg 900
acatcgtaat ccgcagggct tagcattcac tccaaatggt aaattattgc agtctgaaca 960
aggcccaaac tctgacgatg aaattaacct cattgtcaaa ggtggcaatt atggttggcc 1020
gaatgtagca ggttataaag atgatagtgg ctatgcttat gcaaattatt cagcagcagc 1080
caataagtca attaaggatt tagctcaaaa tggagtaaaa gtagccgcag gggtccctgt 1140
gacgaaagaa tctgaatgga ctggtaaaaa ctttgtccca ccattaaaaa ctttatatac 1200
cgttcaagat acctacaact ataacgatcc aacttgtgga gagatgacct acatttgctg 1260
gccaacagtt gcaccgtcat ctgcctatgt ctataagggc ggtaaaaaag caattactgg 1320
ttgggaaaat acattattgg ttccatcttt aaaacgtggt gtcattttcc gtattaagtt 1380
agatccaact tatagcacta cttatgatga cgctgtaccg atgtttaaga gcaacaaccg 1440
ttatcgtgat gtgattgcaa gtccagatgg gaatgtctta tatgtattaa ctgatactgc 1500
cggaaatgtc caaaaagatg atggctcagt aacaaataca ttagaaaacc caggatctct 1560
cattaagttc acctataagg ctaagtaata cagtcgcatt aaaaaaccga tc      1612
<210> 3
<211> 13
<212> DNA
<213> Artificial Sequence
<220>
<223> primer for point mutation
<400> 3
gccaactcaa cgtgaactga atg
<210> 4
<211> 13
<212> DNA
<213> Artificial Sequence
<220>
<223> primer for point mutation
<400> 4
cttaaatctt cgtggaagta ttc
<210> 5
<211> 13
<212> DNA
<213> Artificial Sequence
<220>
<223> primer for point mutation
<400> 5
ccaagttttc gcggggtggt tag

## Description

### Technical Field

The present invention relates to a modified glucose dehydrogenase in which a specific amino acid residue of a glucose dehydrogenase (GDH) having pyrroloquinoline quinone (PQQ) as a coenzyme is replaced with another amino acid residue. The modified enzyme of the present invention is advantageously used for glucose assay in clinical diagnosis and food analysis.

### Background Art

Blood glucose level is crucial in clinical diagnosis as an important marker for diabetes. In fermentative production using microorganisms, determination of glucose concentration is an important part of the process monitoring. Glucose level is conventionally measured by an enzymatic method using glucose oxidase (GOD) or glucose-6-phosphate dehydrogenase (G6PDH). Application of glucose dehydrogenases having pyrroloquinoline quinone as a coenzyme (PQQGDH) has recently come to attention. PQQGDHs have high oxidation activity for glucose, and they do not require oxygen as an electron acceptor because they have a coenzyme bonded thereto. Therefore, PQQGDHs are expected to be used in applications to assay, including a sensing element of a glucose sensor.

PQQGDHs are glucose dehydrogenases having pyrroloquinoline quinone as a coenzyme and which catalyze the reaction in which glucose is oxidized to gluconolactone. PQQGDHs are known to include membrane-bound enzymes and water-soluble enzymes. Membrane-bound PQQGDHs are single peptide proteins having a molecular weight of about 87 kDa and are widely found in various gram-negative bacteria. On the other hand, water-soluble PQQGDHs have been identified in several strains of Acinetobacter calcoaceticus (Biosci. Biotech. Biochem. (1995), 59 (8), 1548-1555), and the structural gene was cloned and the amino acid sequence was reported (Mol. Gen. Genet. (1989), 217:430-436). A water-soluble PQQGDH derived from A. calcoaceticus is a homodimer composed of two subunits with a molecular weight of about 50 kDa, and requires PQQ and Ca²⁺ for its activity. It exhibits a high enzyme activity in the range of 2,200 to 7,400 U/mg. It has been known that water-soluble PQQGDH is a basic protein whose isoelectric point is about 9.2 in an apoenzyme form, which is not bonded to PQQ, and about 10.2 in a holoenzyme form (K. Matsushita et al. (1995), Biosci. Biotech. Biochem., 59, 1548-1555). A result of X-ray structural analysis of the water-soluble PQQGDH was recently reported to reveal the three-dimensional structure and presumed positions of PQQ and Ca²⁺ (A. Oubrie et al. (1999), J. Mol. Bio., 289, 319-333; A. Oubrie et al. (1999), The EMBO Journal, 18 (19), 5187-5194).

With regard to purification of water-soluble PQQGDHs, Duine et al. reported complete purification of a water-soluble PQQGDH from A. calcoaceticus with a specific activity of 640 U/mg at 10% yield (P. Dokter et al. (1986) Biochem. J., 239, 163-167). Water-soluble fractions prepared from cells of A. calcoaceticus were subjected to cation-exchange chromatography, gel filtration chromatography, cation-exchange chromatography, and gel filtration chromatography, in that order, and a single band of about 50 kDa by SDS-PAGE was identified. In a following study, they achieved a specific activity of 2,214 U/mg at 44% yield (K. Matsushita et al., supra). In addition, they introduced the structural gene of a water-soluble PQQGDH into Escherichia coli, and obtained recombinant PQQGDH with a specific activity of 7,400 U/mg at 41% yield through two times of cation-exchange chromatography and hydrophobic chromatography (A. J. J. Olsthoorn, and J. A. Duine (1996), Archives of Biochem. Biophys., 336, 42-48).

For efficient PQQGDH production, some recombinant processes have been reported which utilize Escherichia coli, yeast or enterobacteria as a host. A water-soluble PQQGDH obtained by such recombinant processes is expressed as a basic water-soluble protein with a very high isoelectric point. Accordingly, its purification is principally performed with cation-exchange chromatography. However, it is difficult to remove other basic proteins derived from the host by only cation-exchange chromatography.

A process for purifying basic proteins has been known in which an arginine tail is added as an affinity tail to a C-terminus to enhance the affinity for a cation exchange column (H. M. Sassenfeld and S. J. Brewe (1984) Biotechnology, 2, 76-81). It is expected that the application of this process to a water-soluble PQQGDH, which is a basic protein, would increase the surface charge of the water-soluble PQQGDH to enhance the affinity for cation exchange columns. Unfortunately, if such a protein bearing an arginine tail is produced in Escherichia coli, the arginine residue is cleaved at the C-terminus due to the outer-membrane protease of the Escherichia coli. Thus, it is disadvantageously difficult to obtain a pure enzyme sample.

Accordingly, the object of the present invention is to provide a modified water-soluble PQQGDH which allows efficient recovery of the recombinant PQQGDH.

### Disclosure of Invention

The inventors of the present invention have conducted intensive research to develop a modified PQQGDH capable of being easily purified by modifying a known water-soluble PQQGDH. As a result, they have successfully obtained a modified enzyme by substituting arginine for a specific residue located at the surface of a water-soluble PQQGDH. Such a modified enzyme can be easily purified by cation-exchange chromatography.

The present invention provides a modified glucose dehydrogenase comprising a water-soluble glucose dehydrogenase having a pyrroloquinoline quinone as a coenzyme, wherein arginine is substituted for at least one amino acid residue present on the surface of the molecule, selected from the group consisting of glutamine, asparagine, and threonine.

Preferably, the water-soluble glucose dehydrogenase having a pyrroloquinoline quinone as a coenzyme is a water-soluble PQQGDH derived from Acinetobacter calcoaceticus.

The present invention also provides a modified glucose dehydrogenase comprising a water-soluble glucose dehydrogenase having a pyrroloquinoline quinone as a coenzyme, derived from Acinetobacter calcoaceticus, wherein arginine is substituted for at least one amino acid residue selected from the group consisting of glutamine 209, asparagine 240, and threonine 389. More preferably, the glutamine 209, the asparagine 240, and the threonine 389 are replaced with arginine.

The present invention also provides a gene encoding the modified glucose dehydrogenase of the invention, a vector comprising the gene, a transformant containing the gene, and a glucose assay kit and a glucose sensor comprising the modified glucose dehydrogenase of the present invention.

### Brief Description of the Drawings

Fig. 1 shows a process for preparing a mutant gene encoding a modified enzyme of the present invention.
Fig. 2 shows the structure of a plasmid pGB2 used in the present invention.
Fig. 3 is an SDS-PAGE result of a modified enzyme of the present invention.
Fig. 4 shows the enzyme activities of chromatography fractions of the modified enzyme of the present invention.
Fig. 5 shows a glucose assay using a modified PQQGDH of the present invention.
Fig. 6 shows a calibration curve of an enzyme sensor comprising a modified PQQGDH of the present invention.

### Best Mode for Carrying Out the Invention

All the descriptions of patent documents and other references explicitly referred to herein are incorporated herein by reference. The entire description of Japanese Patent Application No. 2001-294846, on the basis of which the present application claims a priority, is also incorporated herein by reference.

### Design of modified PQQGDH

In order to prepare a modified PQQGDH of the present invention, a target site of a wild-type water-soluble PQQGDH is selected according to the three-dimensional information, which allows amino acid substitution to increase the surface charge without varying the enzyme activity and other characteristics, such as stability. The selection is conducted according to the following guidelines: the site is present on the surface of the water-soluble PQQGDH protein; the site is a neutral residue; the site is a polar residue; the side chain of the site is exposed at the surface of the molecule and is not expected to substantially interact with other residues; and the site is present in a region that is probably not an enzyme active site or a substrate binding site. By substituting a basic residue, particularly an arginine residue, for an amino acid residue thus selected, the surface charge of the protein is increased without largely affecting the enzyme activity. Preferably, the amino acid residue to be modified is selected from the group consisting of glutamine, asparagine, and threonine.

Since the surface charge of the resulting modified PQQGDH is increased, the modified PQQGDH tightly bonds to cation-exchange chromatography. Accordingly, the modified PQQGDH can be easily separated and purified from the other proteins derived from the host by cation-exchange chromatography.

In the modified glucose dehydrogenase of the present invention, some of the other amino acid residues may be deleted or replaced, or another amino acid residue may be added, as long as the modified glucose dehydrogenase has a desired glucose dehydrogenase activity.

Those skilled in the art can also obtain a PQQGDH with an increased surface charge from a water-soluble PQQGDH derived from other bacteria by substituting an arginine residue for a neutral basic amino acid residue present at the surface of the molecule, according to the teaching of the present invention.

### Process for preparing modified PQQGDH

The sequence of a gene encoding the wild-type water-soluble PQQGDH derived from Acinetobacter calcoaceticus is defined by SEQ ID NO:2.

A gene encoding the modified PQQGDH of the present invention can be constructed by substituting the base sequence encoding an amino acid residue to be replaced in the gene encoding a wild-type water-soluble PQQGDH with a base sequence encoding arginine. Various techniques for such site-specific base sequence substitution are known in the art, as described in, for example, Sambrook et al., "Molecular Cloning: A Laboratory Manual", Second Edition, 1989, Cold Spring Harbor Laboratory Press, New York.

The resulting mutant gene is inserted into a gene expression vector (for example, a plasmid) to transform into an appropriate host (for example, Escherichia coli). A large number of vector/host systems for expressing a foreign protein are known, and various organisms, such as bacteria, yeasts, and cultured cells, may be used as a host.

The resulting transformant expressing a modified PQQGDH is cultured and collected by centrifugation or other means from the culture medium, and then disrupted by a French press or by means of osmotically shock to release the periplasmic enzyme into the medium. The sample is ultracentrifuged to yield a water-soluble fraction containing PQQGDH. Alternatively, the expressed PQQGDH may be secreted into the medium using an appropriate host/vector system.

Then, the resulting water-soluble fraction is purified by cation-exchange chromatography. The purification can be performed in accordance with the instructions of textbooks generally known in the art. Various types of cation-exchange chromatography columns for protein purification are known in the art, and any of these columns may be used in the present invention. Exemplary columns include CM-5PW, CM-Toyopearl 650M, and SP-5PW (Tosoh Corp.) and S-sepharose, Mono-S, and S-Resorce (Pharmacia Inc.). A column is equilibrated with an appropriate buffer solution and a sample is loaded to the column to wash out non-adsorbed constituents. Exemplary buffers include phosphate buffers and MOPS buffers.

Then, the constituents adsorbed to the column are eluted using a buffer with a higher salt concentration. The salt concentration can be varied by using plural buffers with different salt concentrations sequentially or by providing a linear gradient of salt concentration, or in combination thereof. The elution of the sample is monitored by absorptiometry or the like, and the eluent is fractionated in an appropriate volume. The enzyme activity of each fraction is measured and a desired fraction is collected to yield the modified enzyme of the present invention in a purified form.

In addition, another protein purification process known in the art, such as filtration, dialysis, gel filtration chromatography, or affinity chromatography, may be applied, if necessary, before or after cation-exchange chromatography.

The purity of the protein is determined by a method known in the art, such as SDS-PAGE or HPLC.

### Method for measuring enzyme activity

The PQQGDH of the present invention, associating with PQQ as a coenzyme, catalyzes a reaction in which glucose is oxidized to gluconolactone. The enzyme activity can be measured by determining the quantity of PQQ reduced concurrently with a PQQGDH-catalyzed glucose oxidation usinga color-developing reaction of a redox dye. Exemplary color-developing reagents include PMS (phenazine methosulfate)-DCIP(2,6-dichlorophenylindophenol), potassium ferricyanide, and ferrocene.

### Glucose assay kit

The present invention is also directed to a glucose assay kit containing the modified PQQGDH according to the present invention. The glucose assay kit of the present invention contains a modified PQQGDH according to the present invention in an amount sufficient for at least one run of assay. In addition to the modified PQQGDH of the present invention, the kit typically contains a buffer necessary for the assay, a mediator, standard glucose solutions for preparing a calibration curve, and instructions for use. The modified PQQGDH of the present invention may be provided in various forms, such as freeze-dried reagents and solutions in appropriate preservative solutions. Preferably, the modified PQQGDH of the present invention is provided in the form of a holoenzyme, but it may be provided in the form of apoenzyme and converted into a holoenzyme before use.

### Glucose sensor

The present invention is also directed to a glucose sensor using the modified PQQGDH according to the present invention. Suitable electrodes include carbon, gold, and platinum electrodes, and on which the enzyme of the present invention is immobilized. Immobilization is conducted by using a crosslinking agent; encapsulation in a polymer matrix; coating with a dialysis membrane; using a photocrosslinkable polymer, an electrically conductive polymer, or a redox polymer; or fixing in a polymer or adsorbing onto the electrode together with an electron mediator such as ferrocene and its derivatives. These methods may be applied in combination. Preferably, the modified PQQGDH of the present invention is immobilized on an electrode in the form of a holoenzyme, but it may be immobilized in the form of apoenzyme and PQQ may be provided in a separate layer or solution. Typically, the modified PQQGDH of the present invention is immobilized on a carbon electrode with glutaraldehyde, then treated with a reagent containing an amine group to block the free radical of glutaraldehyde.

Glucose concentration is measured as below. PQQ, CaCl₂, and a mediator are added to a thermostatic cell containing a buffer and maintain the temperature constant. Exemplary mediators include potassium ferricyanide and phenazine methosulfate. The electrode on which the modified PQQGDH has been immobilized is used as a working electrode, in combination with a counter electrode (for example, platinum electrode) and a reference electrode (for example, Ag/AgCl electrode). After a constant voltage is applied to the carbon electrode to reach a steady current, a sample containing glucose is added and the increase in current is measured. The glucose concentration in the sample can be calculated from the calibration curve prepared with glucose standard solutions.

### Examples

The present invention will be further illustrated with reference to examples, without limiting the scope of the invention.

### Example 1 Construction of modified PQQGDH gene:

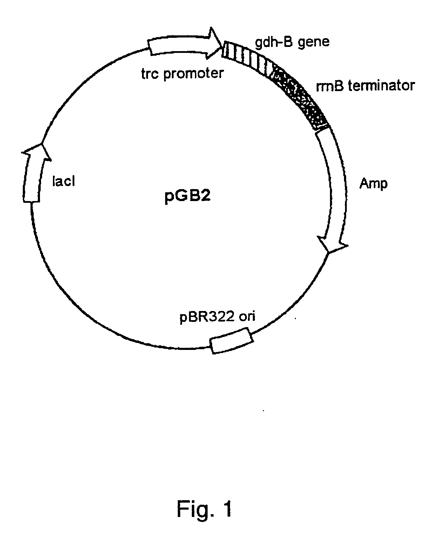

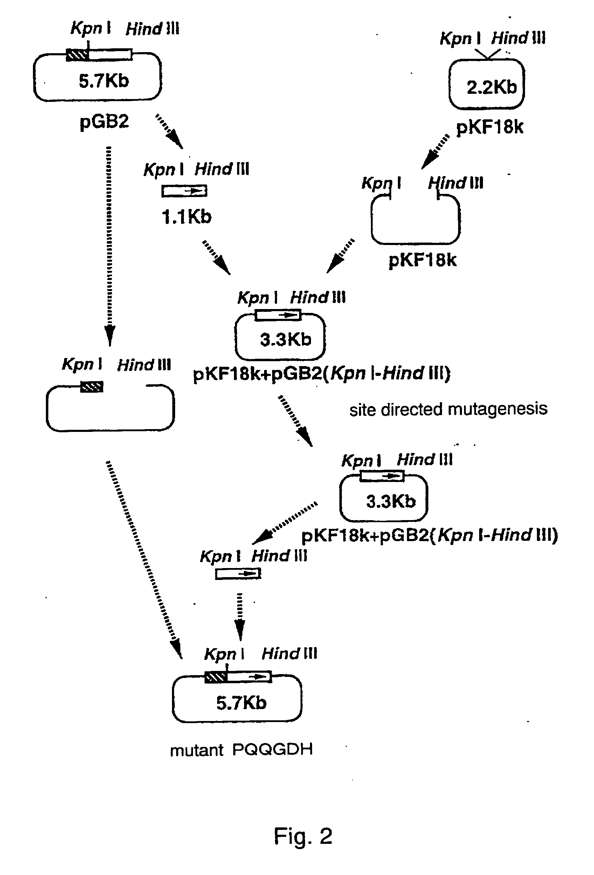

A modification was introduced into the structural gene of a PQQGDH derived from Acinetobacter calcoaceticus shown in SEQ ID NO:2. A plasmid pGB2 is prepared by inserting the structural gene encoding a PQQGDH derived from Acinetobacter calcoaceticus into the multicloning site of a vector pTrc99A (Pharmacia Inc.) (Fig. 2). Nucleotide sequences encoding glutamine 209, asparagine 240, and threonine 389 were replaced with a base sequence encoding arginine by site-directed mutagenesis in the usual manner. The side-directed mutagenesis was performed using the plasmid pGB2 by the method shown in Fig. 1. The sequence of the synthetic oligonucleotide target primer used in the mutagenesis is as follows:

Q209R 5'-GCCAACTCAACGTGAACTGAATG-3' (SEQ ID NO:3)

D229R 5'-CTTAAATCTTCGTGGAAGTATTC -3' (SEQ ID NO:4)

N240R 5'-CCAAGTTTTCGCGGGGTGGTTAG-3' (SEQ ID NO:5)

A Kpn I-Hind III fragment containing part of the gene encoding the PQQGDH derived from Acinetobacter calcoaceticus was inserted into a vector plasmid pKF18K (Takara Shuzo Co., Ltd.) to prepare a template. Fifty femto moles of this template, 5 pmol of the selection primer attached to the Mutan™ -Express Km Kit (Takara Shuzo Co., Ltd.), and 50 pmol of a phosphorylated target primer were mixed with the annealing buffer attached to the same kit in an amount one-tenth the total volume (20 µl), and heated at 100°C for 3 minutes to denature the plasmid into a single strand. The selection primer serves to recover a dual amber mutation on the kanamycin-resistant gene of pKF18k. The mixture was placed on ice for 5 minutes to anneal the primers. To this mixture were added 3 µL of the extension buffer attached to the same kit, 1 µL of T4 DNA ligase, 1 µL of T4 DNA polymerase, and 5 uL of sterilized water for synthesizing a complementary strand.

A DNA mismatch repair-deficient strain, E. coli MBH 71-18 mutS was transformed with the plasmid, and shake-cultured overnight to amplify the plasmid.

Then, the plasmid extracted from the culture was transformed into E. coli MV1184, and a plasmid was prepared from a colony. The resulting plasmid was sequenced to confirm that the intended mutation had been introduced. The fragment of the plasmid was substituted for the Kpn I-Hind III fragment of the gene encoding the wild-type PQQGDH on the plasmid pGB2 to construct a gene coding for a modified PQQGDH having three mutations of Q209R, D229R, and N240R (hereinafter referred to as the modified PQQGDH).

### Example 2 Preparation of modified enzyme:

The gene encoding the wild-type or modified PQQGDH was inserted into the multicloning site of an E. coli expression vector pTrc99A (Pharmacia Inc.), and the resulting plasmid was transformed into the Escherichia coli strain DH50α. The transformant was shake-cultured at 37°C overnight on 450 mL of L medium (containing 50 pg/L of ampicillin) in a Sakaguchi flask, and inoculated in 7 L of L medium containing 1 mM of CaCl₂ and 500 µM of PQQ. About 3 hours after starting cultivation, isopropylthiogalactoside was added at a final concentration of 0.3 mM, and the cultivation was continued for another 1.5 hours. The cultured cells were collected by centrifugation (5,000 × g, 10 min, 4°C) and washed twice with a 0.85% NaCl solution. The cells were suspended in a 10 mM phosphate buffer (pH 7.0), and disrupted with a French press (110 MPa). Undisrupted cells were removed by two times of centrifugation (15,000 × g, 15 min, 4°C). The supernatant was ultracentrifuged (40,000 r.p.m., 90 min, 4°C) to yield a water-soluble fraction. The fraction was dialyzed with buffer A (10 mM MOPS-NaOH buffer (pH 7.0) at 4°C overnight to yield a crude fraction.

### Example 3 Purification by cation-exchange chromatography:

The crude fraction prepared in Example 2 was filtrated through a 0.2 µm filter before applying to the column. Cation-exchange chromatography was performed using CM-5PW column (Tosoh Corp.), a 10 mM MOPS-NaOH buffer (pH 7.0) as buffer A, and a 0.8 M NaCl + 10 mM MOPS-NaOH buffer (pH 7.0) as buffer B.

First, the column was equilibrated with buffer A. After adsorbing the sample, the column was washed with buffer A in an amount 5 times the column volume. Then, the sample was subjected to a linear gradient of 0 to 0.64 M of NaCl (120 min) using buffer B to elute a targeted enzyme. The flow rate was 0.5 mL/min. The eluted protein was detected at an absorption wavelength of 280 nm. Aliquots of the eluent were collected in every 2 minutes The wild-type water-soluble PQQGDH showed a peak of elution at a salt concentration of about 80 mM at about 20 minutes in cation-exchange chromatography; while the modified water-soluble PQQGDH showed a peak at a salt concentration of about 190 mM at about 38 minutes.

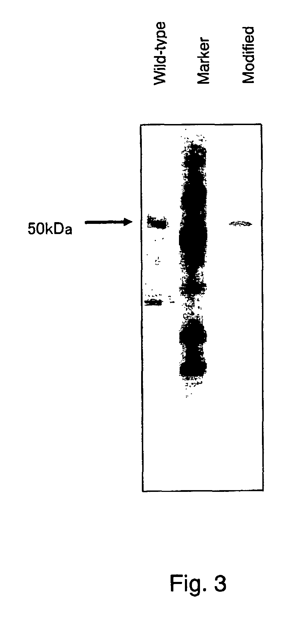

Fig. 3 shows the results of SDS-PAGE analysis of the peak fractions. The modified water-soluble PQQGDH showed a single band with the intended molecular weight of 50 kDa. Thus, even this one run of chromatography was able to achieve nearly complete purification. In contrast, the wild-type water-soluble PQQGDH showed some bands of contaminants.

### Example 4 Measurement of enzyme activity:

Enzyme activity was measured in a 10 mM MOPS-NaOH buffer (pH 7.0) using PMS (phenazine methosulfate)-DCIP(2,6-dichlorophenylindophenol) by monitoring changes in the absorbance of DCIP at 600 nm with a spectrophotometer. The rate of decrease in absorbance was defined as the reaction rate of the enzyme. In this instance, the enzyme activity for reducing 1 µmol of DCIP in 1 minute was defined as 1 unit. The molar absorption coefficient of DCIP at pH 7.0 was 16.3 mM⁻¹.

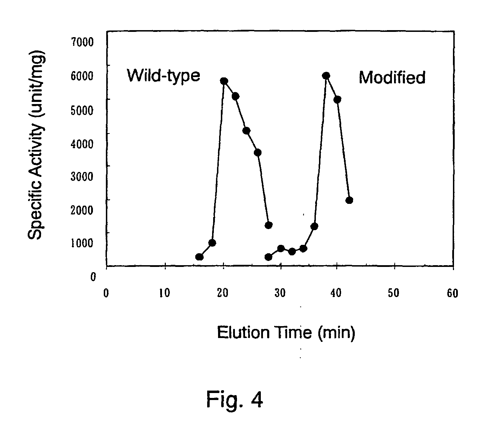

The enzyme activities of the fractions obtained by chromatography are shown in Fig. 4. The horizontal axis represents elution time, and the vertical axis represents GDH activity.

### Example 5 Evaluation of enzyme activity and substrate specificity:

The active fraction, non-adsorbed fraction, and crude fraction obtained by chromatography were dialyzed at 4°C with a 10 mM MOPS-NaOH buffer (pH 7.0) in an amount 100 times the amount of the fractions, and converted into holoenzymes in the presence of 1 µM of PQQ and 1 mM of CaCl₂ for 1 hour or more. These fractions were divided into aliquots of 187 µL. To each aliquot were added 3 µL of an activating agent (48 µL of 6 mM DCIP; 8 µL of 600 mM PMS; 16 µL of 10 mM phosphate buffer pH 7.0) and 10 µL of a substrate selected from 20 mM glucose, 2-deoxy-D-glucose, mannose, allose, 3-o-methyl-D-glucose, galactose, xylose, lactose, and maltose. The aliquots were measured for the enzyme activity at room temperature by the method shown in Example 4. The Km and Vₘₐₓ were determined from the plot of substrate concentration vs. enzyme activity.

The activity of the wild-type water-soluble PQQGDH for glucose was about 7,100 U/mg; the activity of the modified water soluble PQQGDH was about 7,800 U/mg. Thus, both showed substantially the same activity. The wild-type and modified water-soluble PQQGDHs showed substantially the same Km and Vₘₐₓ values for substrates other than glucose, suggesting that introduction of mutation did not change the substrate specificity.

### Example 6 Glucose assay:

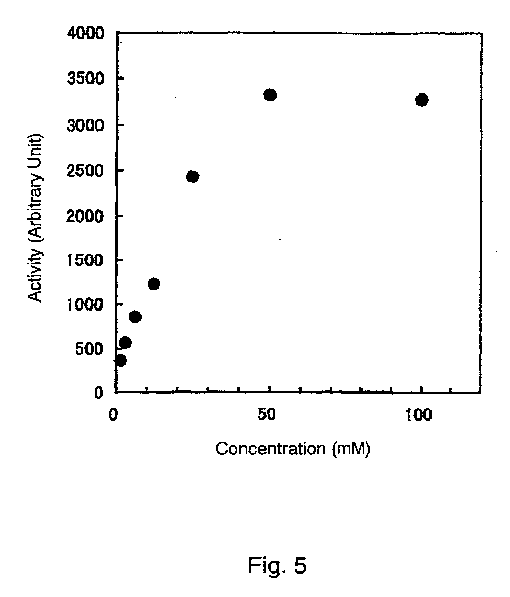

The modified PQQGDH was used for assaying glucose. The modified enzyme was converted into a holoenzyme in the presence of 1 pM of PQQ and 1 mM of CaCl₂ for 1 hour or more, and measured for the enzyme activity in the presence of various concentrations of glucose, 5 µM of PQQ, and 10 mM of CaCl₂. The measurement was performed according to the method in Example 4 with reference to the changes in absorbance of DCIP at 600 nm. As shown in Fig. 5, the modified PQQGDH can be used for assaying glucose in the range of 5 to 50 mM.

### Example 7 Preparation and evaluation of enzyme sensor:

Five units of the modified enzyme were freeze-dried with 20 mg of carbon paste. After thorough mixing, the mixture was applied only on the surface of a carbon paste electrode preliminarily filled with about 40 mg of carbon paste and polished on a filter paper. This electrode was treated in a 10 mM MOPS buffer (pH 7.0) containing 1% of glutaraldehyde at room temperature for 30 minutes, and then in a 10 mM MOPS buffer (pH 7.0) containing 20 mM of lysine at room temperature for 20 minutes to block glutaraldehyde. The electrode was equilibrated in a 10 mM MOPS buffer (pH 7.0) at room temperature for 1 hour or more. The electrode was stored at 4°C.

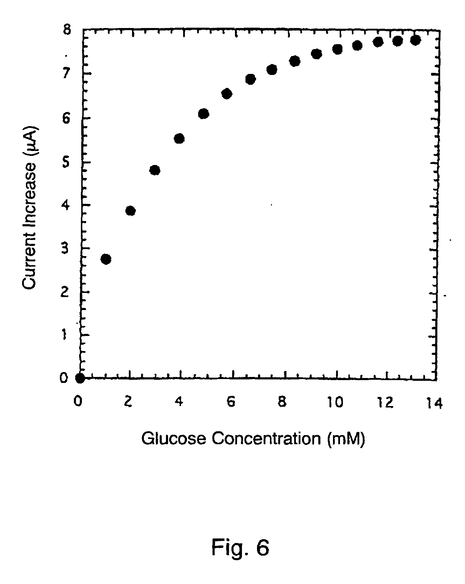

Glucose concentration was measured with the resulting glucose sensor. Fig. 6 shows a calibration curve of the sensor. As shown in the figure, the modified PQQGDH can be used for the determination of glucose in the range of 1 to 12 mM.

### Industrial Applicability

The present invention provides a modified water-soluble PQQGDH which allows efficient recovery of the recombinant PQQGDH. The modified enzyme of the present invention is advantageously used for glucose assay in clinical diagnosis and food analysis.

## Claims

1. A modified glucose dehydrogenase comprising a water-soluble glucose dehydrogenase having a pyrroloquinoline quinone as a coenzyme, wherein arginine is substituted for at least one amino acid residue present on the surface of the molecule thereof, selected from the group consisting of glutamine, asparagine, and threonine.

2. A modified glucose dehydrogenase according to Claim 1, wherein the water-soluble glucose dehydrogenase having a pyrroloquinoline quinone is a water-soluble PQQGDH derived from Acinetobacter calcoaceticus.

3. A modified glucose dehydrogenase comprising a water-soluble glucose dehydrogenase having a pyrroloquinoline quinone as a coenzyme, derived from Acinetobacter calcoaceticus, wherein arginine is substituted for at least one amino acid residue selected from the group consisting of glutamine 209, asparagine 240, and threonine 389.

4. A modified glucose dehydrogenase according to Claim 3, wherein the glutamine 209, the asparagine 240, and the threonine 389 are replaced with arginine.

5. A gene encoding the modified glucose dehydrogenase as set forth in any one of Claims 1 to 4.

6. A vector containing the gene as set forth in Claim 5.

7. A transformant containing the gene as set forth in Claim 5.

8. An organism containing the gene as set forth in Claim 5, integrated into the main chromosome thereof.

9. A method for preparing the modified glucose dehydrogenase as set forth in any one of Claims 1 to 4 using the organism as set forth in Claim 8.

10. A glucose assay kit comprising the modified glucose dehydrogenase as set forth in any one of Claims 1 to 4.

11. A glucose sensor comprising the modified glucose dehydrogenase as set forth in any one of Claims 1 to 4.
